# DEMANDE DE BREVET EUROPEEN

(11) **EP 1 312 348 A2**
(43) Date de publication de la demande: **21.05.2003**
(21) Numéro de dépôt: 02292628.1
(22) Date de dépôt: 23.10.2002
(51) Int. Cl.: A61K 7/135, A61K 7/06, C08L 83/08

(54) **Utilisation de silicones aminées particulières en pré- ou post-traitement de décoloration de fibres kératiniques**

(30) Priorité: 08.11.2001 FR 0114471
(71) Demandeur: L'OREAL, 75008 Paris (FR)
(72) Inventeur: Devin-Baudoin, Priscille, 92170 Vanves (FR); Sabbagh, Anne, 92500 Rueil Malmaison (FR)
(74) Mandataire: Wattremez, Catherine

(57) **Abrégé**

La présente invention a pour objet l'utilisation, en pré- ou en post- traitement d'une décoloration des fibres kératiniques humaines et plus particulièrement des cheveux, d'une composition comprenant au moins une silicone aminée particulière.

Elle a aussi pour objet des procédés de décoloration des fibres kératiniques humaines et plus particulièrement dès cheveux comprenant un pré- ou un post- traitement avec une composition comprenant au moins une silicone aminée particulière.

## Description

La présente invention a pour objet l'utilisation, en pré- ou en post- traitement d'une décoloration des fibres kératiniques humaines et plus particulièrement des cheveux, d'une composition comprenant au moins une silicone aminée particulière.
Elle a aussi pour objet des procédés de décoloration des fibres kératiniques humaines et plus particulièrement des cheveux comprenant un pré- ou un post- traitement avec une composition comprenant au moins une silicone aminée particulière.

Les nuances naturelles des cheveux foncés peuvent être éclaircies de façon durable au moyen de traitements de décoloration. Il en va de même pour les nuances artificiellement communiquées aux cheveux par le biais des teintures directes ou d'oxydation et que l'on désire effacer.
Les compositions de décoloration utilisées dans ces traitements sont constituées de compositions aqueuses épaissies de peroxyde d'hydrogène, prêtes à l'emploi. Mais elles se présentent principalement sous forme de produits anhydres ( poudres ou crèmes ) contenant des composés alcalins (amines et silicates alcalins), et un réactif oxydant peroxygéné tels que les persulfates, perborates ou percarbonates, d'ammonium ou de métaux alcalins, que l'on dilue au moment de l'emploi avec une composition aqueuse de peroxyde d'hydrogène.

Ces traitements très efficaces induisent cependant une sensibilisation des cheveux ; les cheveux deviennent plus secs, plus difficiles à démêler, avec un toucher rêche.
D'autres traitements de décoloration utilisent des compositions prêtes à l'emploi constituées de produits anhydres (poudres ou crèmes) comprenant des agents réducteurs qui sont mélangés au moment de l'emploi avec une composition aqueuse contenant éventuellement un agent de pH. Ces traitements réducteurs peuvent également induire une sensibilisation des cheveux.

Jusqu'ici, pour améliorer l'état de la fibre capillaire après un traitement de décoloration, on a recours à des soins rincés ou non rincés, tels que des après-shampooings, des masques traitants, des crèmes traitantes, ou des sérums.
Ces soins ont l'inconvénient d'être temporaires et doivent être renouvelés après chaque lavage des cheveux.
En outre, la plupart du temps, ils alourdissent les cheveux, les rendant mous, avec un toucher enrobé glissant peu naturel.

Il existe donc un besoin d'améliorer l'état que présente les cheveux après un traitement de décoloration.

Après d'importantes études sur la question, la demanderesse a découvert de manière tout à fait inattendue et surprenante que l'utilisation, en pré- ou post- traitement sur des fibres kératiniques humaines et plus particulièrement des cheveux, d'une composition comprenant au moins une silicone aminée particulière, permettait de résoudre ce problème.
Cette découverte est à l'origine de la présente invention.

Un premier objet de l'invention concerne donc l'utilisation, en pré- ou post- traitement d'une décoloration des fibres kératiniques humaines et plus particulièrement des cheveux, d'une composition comprenant au moins une silicone aminée aminoéthyliminoalkyl(C₄-C₈).

Ledit procédé a notamment pour objet d'améliorer l'état des cheveux après décoloration, les cheveux sont plus doux, lisses de la racine à la pointe, individualisés, légers, souples et soyeux. Par ailleurs, ils offrent un toucher bien plus naturel qu'avec un agent conditionneur de l'art antérieur utilisé après une décoloration. En outre, ils se démêlent bien et se coiffent bien plus facilement. Le traitement selon l'invention a pour autre avantage de ne pas modifier le pouvoir éclaircissant du traitement de décoloration et les effets cosmétiques ainsi produits sont durables et visibles pendant au moins six semaines.

Par amélioration de l'état de la fibre on entend une diminution de la porosité ou de la solubilité alcaline de la fibre et une amélioration des propriétés cosmétiques et en particulier du lissage, de la douceur et de la facilité de démêlage et de coiffage.
Cet effet est rémanent, c'est à dire durable.
La porosité se mesure par la fixation à 37°C et à pH10, en 2 minutes, de la 2-nitro paraphénylènediamine à 0,25% dans un mélange éthanol / tampon pH 10 (rapport volumique 10/90).
La solubilité alcaline correspond à la perte de masse d'un échantillon de 100 mg de fibres kératiniques sous l'action de la soude décinormale pendant 30 minutes à 65°C.

Un second objet de l'invention concerne un procédé de décoloration consistant à appliquer sur les fibres kératiniques humaines et plus particulièrement les cheveux, humides ou secs, lavés ou non lavés, une composition comprenant au moins une silicone aminée aminoéthyliminoalkyl(C₄-C₈), à la laisser agir à température ambiante ou avec apport de chaleur, à rincer ou ne pas rincer les fibres, puis à appliquer la composition décolorante, à laver les fibres, les rincer puis les sécher.

Un autre objet de l'invention concerne un procédé de décoloration consistant à appliquer sur les fibres kératiniques humaines et plus particulièrement les cheveux, une composition décolorante, à les laver au shampooing, à les rincer à l'eau, puis à appliquer sur les fibres humides ou sèches, une composition comprenant au moins une silicone aminée aminoéthyliminoalkyl(C₄-C₈), à la laisser agir à température ambiante ou avec apport de chaleur, à rincer ou ne pas rincer les fibres, puis à les sécher. Selon ce procédé de post- traitement, la composition peut être appliquée immédiatement après décoloration ou de manière différée, et de façon répétitive.

### Silicones aminées

Les silicones aminées selon l'invention présentent la formule suivante : 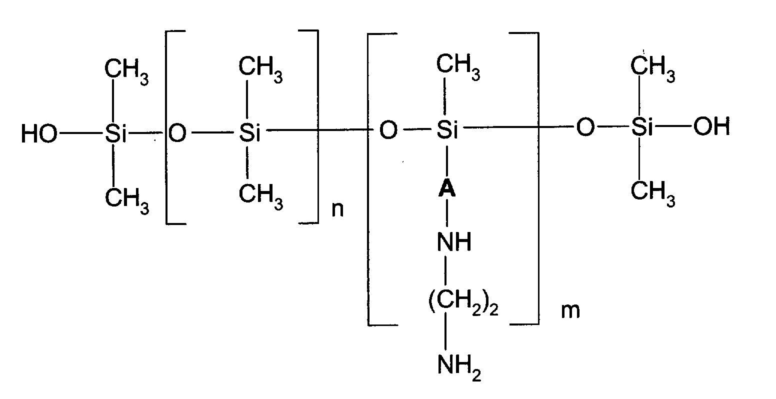 dans laquelle :
**A** désigne un radical alkylène linéaire ou ramifié en C₄-C₈ et de préférence en C₄
m et n sont des nombres tels que la somme (n + m) peut varier notamment de 1 à 2 000 et en particulier de 50 à 150, n pouvant désigner un nombre allant de 0 à 1 999 et notamment de 49 à 149 et m pouvant désigner un nombre allant de 1 à 2 000, et notamment de 1 à 10.

On entend par radical alkylène des groupements hydrocarbonés saturés divalents.

La viscosité du polymère est de préférence supérieure à 25 000 mm²/s à 25°C.
Plus préférentiellement, cette viscosité peut aller de 30 000 à 200 000 mm²/s à 25°C et encore plus préférentiellement de 30 000 à 150 000 mm²/s à 25°C.
La viscosité des silicones est par exemple mesurée selon la norme « ASTM 445 Appendice C ».

La silicone aminée présente une masse moléculaire moyenne en poids allant de préférence de 2000 à 1000000 et encore plus particulièrement de 3500 à 200000.
Les masses moléculaires moyennes en poids de ces silicones aminées sont mesurées par Chromatographie par Perméation de Gel (GPC) à température ambiante en équivalent polystyrène. Les colonnes utilisées sont des colonnes µ styragel. L'éluant est le THF, le débit est de 1 ml/mn. On injecte 200 µl d'une solution à 0,5% en poids de silicone dans le THF. La détection se fait par réfractométrie et UVmétrie.

Lorsque ces silicones aminées sont mises en oeuvre, une forme de réalisation particulièrement intéressante est leur utilisation sous forme d'émulsion huile-dans-eau. L'émulsion huile-dans-eau peut comprendre un ou plusieurs tensioactifs. Les tensioactifs peuvent être de toute nature mais de préférence cationique et/ou non ionique.

Les particules de silicone dans l'émulsion ont une taille moyenne allant généralement de 3 nm à 500 nanomètres, de préférence de 5 nm à 300 nanomètres, plus particulièrement de 10 nm à 275 nanomètres et encore plus particulièrement de 150 nm à 275 nanomètres.

Une silicone répondant à cette formule est par exemple la DC2-8299® de la société DOW CORNING.

La silicone aminée est utilisée de préférence dans la composition de pré- ou de post-traitement en une quantité allant de 0,01 à 20% en poids du poids total de la composition. Plus préférentiellement, cette quantité va de 0,1 à 15% en poids et encore plus particulièrement de 0,5 à 10 % en poids.

La composition de pré- ou de post- traitement peut contenir tous les ingrédients classiquement utilisés en cosmétique et en particulier dans le domaine capillaire. En particulier elle peut contenir des tensioactifs et/ou des polymères additionnels. Ces tensioactifs et ces polymères peuvent être de nature non-ionique, cationique, anionique ou amphotère.
Parmi les polymères additionnels, les silicones aminées autres que celles de l'invention sont particulièrement préférées.

La composition de pré- ou de post- traitement présente un pH allant de 2 à 11 et de préférence de 4 à 9.
Elle peut se présenter sous diverses formes telles que lotions, gels, crèmes, shampooings, sticks, mousses, sprays. Pour certaines de ces formes, elle peut être conditionnée en flacon pompe ou dans un récipient aérosol. Dans le cas de l'aérosol, la composition est associée à un agent propulseur qui peut être par exemple un alcane ou un mélange d'alcane, du diméthyl éther, de l'azote, du protoxyde d'azote, du gaz carbonique ou des halgénoalcanes, ainsi que leurs mélanges.
Une forme particulièrement préférée selon l'invention est la forme shampooing.
Dans ce cas, la composition contient au moins un agent tensioactif qui est de préférence anionique. De préférence alors, elle contient un mélange de tensioactifs dont au moins un agent tensioactif anionique, le ou les autres tensioactifs étant préférentiellement non ioniques ou amphotères.

Comme il est indiqué ci dessus, la composition de post- traitement peut être appliquée immédiatement après décoloration ou de manière différée. Par différée, on entend une application se faisant quelques heures, un jour ou plusieurs jours (de 1 à 60 jours) après la décoloration.
De préférence, plusieurs applications sont effectuées entre deux décolorations.
Le nombre d'applications entre deux décolorations est de préférence compris entre 1 et 60 et encore plus préférentiellement entre 2 et 30.

Les compositions de décoloration, comme on l'a déjà dit plus avant sont réductrices ou oxydantes.

Lorsqu'elles sont réductrices, elles se présentent principalement sous forme de compositions prêtes à l'emploi constituées de produits anhydres (poudres ou crèmes) contenant le ou les agents réducteurs que l'on mélange au moment de l'emploi avec une composition aqueuse contenant éventuellement un agent de pH. Elles peuvent aussi se présenter sous forme de compositions prêtes à l'emploi aqueuses contenant le ou les agents réducteurs au pH approprié. Les agents réducteurs sont principalement des thiols tels que la cystéine, l'acide thioglycolique, l'acide thiolactique, leurs sels et leurs esters, la cystéamine et ses sels, l'acide ascorbique, ses sels et ses esters, l'acide érythorbique, ses sels et ses esters, les sulfites et sulfinates comme l'hydroxyméthanesulfinate de sodium.

Lorsqu'elles sont oxydantes, elles comprennent un ou plusieurs agents oxydants comme le peroxyde d'hydrogène ou des composés susceptibles de produire le peroxyde d'hydrogène par hydrolyse, tels que le peroxyde d'urée ou les persels comme les perborates, les percarbonates et les persulfates.
Lesdites compositions de décoloration oxydantes se présentent principalement sous forme de produits anhydres ( poudres ou crèmes ) contenant des composés alcalins (amines et silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène.
ELles peuvent aussi résulter du mélange, au moment de l'emploi, de la poudre anhydre de réactif péroxygéné avec une composition aqueuse contenant les composés alcalins et une autre composition aqueuse contenant le peroxyde d'hydrogène.
Elles se présentent également sous forme de compositions aqueuses épaissies de peroxyde d'hydrogène, prêtes à l'emploi.

La composition de pré- ou de post- traitement peut être utilisée en mode rincé ou en mode non-rincé, c'est à dire que son application est suivie ou non d'un rinçage.
Dans le premier cas, le temps de pose de la composition de pré- ou de post- traitement est compris entre quelques secondes et 60 minutes et de préférence entre 30 secondes et 15 minutes.

La température d'application de la composition de pré- ou de post- traitement peut varier de 10°C à 70°C. De préférence l'application s'effectuera entre 20 et 60°C et plus particulièrement à la température ambiante.

Les exemples suivants sont destinés à illustrer l'invention. Celle-ci n'est cependant pas limitée à ces modes de réalisation.

### EXEMPLES

On a préparé la composition suivante :
(exprimée en grammes de matière active)

| **Composition** | |
|---|---|
| Polydiméthylsiloxane selon l'invention proposé sous la dénomination DC2-8299® par DOW CORNING | 2 |
| Eau déminéralisée q.s.p | 100 |

La composition a été appliquée sur des cheveux naturels châtain :
- 1/ en pré-traitement d'une décoloration oxydante utilisant le produit du commerce PLATIFIZ® de la société L'OREAL ;
- 2/ en post-traitement d'une décoloration oxydante utilisant le produit du commerce PLATIFIZ® de la société L'OREAL.
En pré-traitement, les cheveux ont ainsi été traités avec cette composition pendant 30 minutes à la température de 37°C, rincés à l'eau, décolorés au PLATIFIZ® et rincés de nouveau abondamment avant séchage.
En post-traitement, les cheveux ont subi une décoloration PLATIFIZ®, ont été rincés à l'eau avant d'être traités par la composition, puis rincés de nouveau abondamment avant d'être séchés.
A l'issue de ces traitements, les cheveux ont été plus lisses, plus doux, plus faciles à démêler qu'en l'absence de pré- ou de post-traitement.
En outre, le niveau de décoloration n'a pas été altéré par lesdits traitements selon l'invention.

## Revendications

1. Utilisation, en pré- ou en post- traitement d'une décoloration des fibres kératiniques humaines et plus particulièrement des cheveux, d'une composition comprenant au moins une silicone aminée aminoéthyliminoalkyl(C₄-C₈).

2. Utilisation selon la revendication 1, **caractérisée par le fait que** la silicone aminée est de formule suivante : 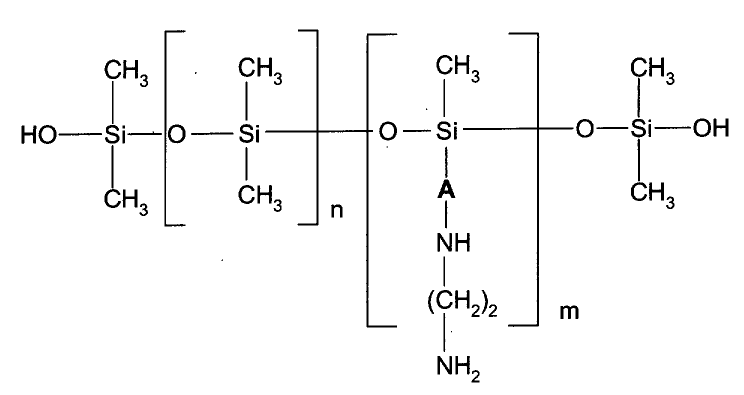 dans laquelle :
**A** désigne un radical alkylène linéaire ou ramifié en C₄-C₈
m et n sont des nombres tels que la somme (n + m) varie de 1 à 2 000 et en particulier de 50 à 150, n désigne un nombre allant de 0 à 1 999 et notamment de 49 à 149 et m désigne un nombre allant de 1 à 2 000, et notamment de 1 à 10.

3. Utilisation selon la revendication 2, **caractérisée par le fait que** A désigne un radical alkylène linéaire ou ramifié en C₄.

4. Utilisation selon l'une quelconque des revendications précédentes, **caractérisée par le fait que** la viscosité du polymère est supérieure à 25 000 mm²/s à 25°C.

5. Utilisation selon la revendication 4, **caractérisée par le fait que** la viscosité va de 30 000 à 200 000 mm²/s à 25°C et plus préférentiellement de 30 000 à 150 000 mm²/s à 25°C.

6. Utilisation selon l'une quelconque des revendications précédentes, **caractérisée par le fait que** la silicone a une masse moléculaire moyenne en poids allant de 2000 à 1000000 et de préférence de 3500 à 200000.

7. Utilisation selon l'une quelconque des revendications précédentes, **caractérisée par le fait que** la silicone aminée est sous forme d'émulsion huile-dans-eau comprenant des agents tensioactifs.

8. Utilisation selon la revendication 7, **caractérisée par le fait que** l'émulsion comprend au moins un agent tensioactif cationique et/ou non ionique.

9. Utilisation selon l'une quelconque des revendications 7 ou 8, **caractérisée par le fait que** les particules de silicone dans l'émulsion ont une taille allant de 3 nm à 500 nanomètres, de préférence de 5 nm à 300 nanomètres, plus particulièrement de 10 nm à 275 nanomètres et encore plus particulièrement de 150 nm à 275 nanomètres.

10. Utilisation selon l'une quelconque des revendications précédentes, **caractérisée par le fait que** la silicone aminée aminoéthyliminoalkyl(C₄-C₈) est présente dans la composition de pré- ou de post- traitement en une quantité allant de 0,01 à 20% en poids du poids total de la composition.

11. Utilisation selon la revendication 10, **caractérisée par le fait qu'**elle est présente en une quantité allant de 0,1 à 15% en poids en poids du poids total de la composition.

12. Utilisation selon la revendication 11, **caractérisée par le fait qu'**elle est présente en une quantité allant de 0,5 à 10 % en poids du poids total de la composition.

13. Utilisation selon l'une quelconque des revendications précédentes, **caractérisée par le fait que** la composition de pré- ou de post- traitement se présente sous forme de lotions, gels, crèmes, shampooings, sticks, mousses, sprays .

14. Utilisation selon l'une quelconque des revendications précédentes, **caractérisée par le fait que** la composition de pré- ou de post- traitement est conditionnée en flacon pompe ou dans un récipient aérosol.

15. Utilisation selon la revendication 14, **caractérisée par le fait que** la composition de pré- ou de post- traitement est associée à au moins un agent propulseur choisi parmi les alcanes, le diméthyl éther, l'azote, le protoxyde d'azote, le gaz carbonique ou les halogénoalcanes.

16. Utilisation selon l'une quelconque des revendications précédentes, **caractérisée par le fait que** la composition de pré- ou de post- traitement comprend au moins un agent tensioactif de nature non-ionique, cationique, anionique ou amphotère.

17. Utilisation selon la revendication 16, **caractérisée par le fait que** la composition de pré- ou de post- traitement comprend un mélange d'agents tensioactifs comprenant au moins un agent tensioactif anionique, le ou les autres agents tensioactifs étant non ioniques ou amphotères.

18. Utilisation selon l'une quelconque des revendications précédentes, **caractérisée par le fait que** la composition de pré- ou de post- traitement comprend au moins un polymère additionnel autre que la silicone aminoéthyliminoalkyl(C₄-C₈).

19. Utilisation selon la revendication 18, **caractérisée par le fait que** le polymère est de nature non-ionique, cationique, anionique ou amphotère.

20. Utilisation selon la revendication 19, **caractérisée par le fait que** le polymère est une silicone aminée différente de la silicone aminoéthyliminoalkyl(C₄-C₈).

21. Utilisation selon l'une quelconque des revendications précédentes, **caractérisée par le fait que** la composition de pré- ou de post- traitement présente un pH allant de 2 à 11 et de préférence de 4 à 9.

22. Utilisation selon la revendication 1 pour améliorer l'état des fibres kératiniques humaines après une décoloration.

23. Procédé de décoloration des fibres kératiniques humaines et plus particulièrement des cheveux, **caractérisé par le fait qu'**il consiste à appliquer sur les fibres, dans une première étape, une composition de pré- traitement comprenant au moins une silicone aminoéthyliminoalkyl(C₄-C₈) et telle que décrite à l'une quelconque des revendications 1 à 9, puis, après avoir rincé ou non rincé les fibres et les avoir séchées ou non séchées, dans une seconde étape, appliquer une composition de décoloration, à laver les fibres, les rincer puis les sécher.

24. Procédé de décoloration des fibres kératiniques humaines et plus particulièrement des cheveux, **caractérisé par le fait qu'**il consiste à appliquer sur les fibres, dans une première étape, une composition décolorante, à la laisser agir pendant un temps suffisant pour décolorer, puis, après avoir rincé ou non rincé les fibres et les avoir séchées ou non séchées, dans une seconde étape, appliquer une composition de post-traitement comprenant au moins une silicone aminoéthyliminoalkyl(C₄-C₈) et telle que décrite à l'une quelconque des revendications 1 à 9, ladite composition de post-traitement étant appliquée, soit immédiatement, soit en différé, et les applications de ladite composition pouvant être répétées entre deux décolorations.

25. Procédé selon l'une quelconque des revendications 23 ou 24, **caractérisé par le fait que** la composition de pré- ou de post- traitement est posée pendant un temps allant de quelques secondes à 60 minutes et de préférence de 30 secondes à 15 minutes.
